# EUROPÄISCHE PATENTANMELDUNG

(11) **EP 0 966 929 A2**
(43) Veröffentlichungstag der Anmeldung: **29.12.1999**
(21) Anmeldenummer: 99105230.9
(22) Anmeldetag: 13.03.1999
(51) Int. Cl.: A61F 2/44

(54) **Implantat zur Fusion zweier Wirbel**

(30) Priorität: 22.06.1998 DE 19827515
(71) Anmelder: Ulrich GmbH & Co. KG, 89081 Ulm (DE)
(72) Erfinder: De Muelenaers, Phillip Francois Rufin Gustaaf, Waterkloofrif 0181 Pretoria (SA)
(74) Vertreter: Hentrich, Swen Dipl.-Phys. Dr.

(57) **Zusammenfassung**

Das Implantat dient für die intersomatische Fusion zweier Wirbel (1) mittels eines anterior/medialen operativen Zugangs und besitzt eine erste Käfigplatte (4) sowie eine über Stützglieder (6) mit der ersten Käfigplatte (4) verbundene zweite Käfigplatte (5), wobei die zweite Käfigplatte (5) kleiner als die erste Käfigplatte (4) ist und die an den Eckkanten der ersten Käfigplatte (4) und der zweiten Käfigplatte (5) angeschlossenen, als Streben (7) ausgebildeten Stützglieder (6) einen Neigungswinkel (alpha) mit den Flächennormalen (N) der ersten Käfigplatte (4) und der zweiten Käfigplatte (5) einschließen.

## Beschreibung

Die Erfindung betrifft ein Implantat für die intersomatische Fusion zweier Wirbel mittels eines anterior/medialen operativen Zugangs, mit einer ersten Käfigplatte sowie einer über Stützglieder mit der ersten Käfigplatte verbundenen zweiten Käfigplatte.

Implantate zur intersomatischen Fusion zweier Wirbel werden genutzt, um nach einer als notwendig erachteten Resektion einer Bandscheibe das betroffene Segment der Wirbelsäule zu stabilisieren. Bei den Operationsverfahren zum Erzielen einer intersomatischen Fusion unterscheidet man zwischen zwei Operationstechniken, nämlich der Posterior Lumbar Interbody Fusion (PLIF) und der Anterior Lumbar Interbody Fusion (ALIF). Aufgrund des unterschiedlichen Zuganges zur Wirbelsäule vom Rücken oder von der Bauchseite her ergeben sich grundsätzlich andere Rahmenbedingungen, die bei der Operation beachtet werden müssen. So müssen bei der PLIF-Technik Ausnehmungen im Wirbelkörper geschaffen werden, durch die das Implantat am Rückenmark vorbei in den Zwischenwirbelraum eingeführt werden kann. Bei der ALIF-Technik sind keine oder nur wesentlich mildere Eingriffe in die Substanz des Wirbelkörpers erforderlich, wobei weiterhin die Gefahr von Beschädigungen von Nerven deutlich reduziert ist.

Die für die PLIF-Technik entwickelten Implantate sind aufgrund des unterschiedlichen Zuganges bei einer intersomatischen Fusion mittels der ALIF-Technik nicht einsetzbar oder bringen nur suboptimale Ergebnisse, da deren Form darauf abgestimmt ist, diese von posterior am Rückenmark vorbei durch die in den Wirbelkörper eingebrachten Aufnahmen in den Zwischenwirbelraum einzuführen, wobei die Implantate in der Regel paarweise rechts und links vom Rückenmark eingesetzt werden müssen.

Der Erfindung liegt daher die Aufgabe zugrunde, ein Implantat der eingangs genannten Art so auszubilden, daß dieses von dem bei der ALIF-Technik zur Verfügung stehenden größeren Platzangebot Gebrauch macht, um eine Stabilisierung des betroffenen Wirbelsäulensegments in einer auf die gesunden Bereiche der Wirbelsäule abgestimmten Lage zu erreichen.

Diese Aufgabe wird nach der Erfindung dadurch gelöst, daß die zweite Käfigplatte kleiner als die erste Käfigplatte ist, und daß die an den Eckkanten der ersten Käfigplatte und der zweiten Käfigplatte angeschlossenen, als Streben ausgebildeten Stützglieder einen Neigungswinkel mit den Flächennormalen der ersten Käfigplatte und der zweiten Käfigplatte einschließen.

Dieses Implantat bietet den Vorteil, daß es als offenes Gerüst konzipiert ist, das eine schnelle und ausreichende Anfangsstabilisierung gewährleistet, darüberhinaus aber auch ein schnelles Einwachsen ermöglicht, da der Innenraum des Implantates nicht von dem Außenraum abgeschottet ist, sondern zwischen den Streben des Implantats hindurch einen Kontakt mit körpereigenem Gewebe bietet, so daß beispielsweise in den Innenraum eingebrachte Knochensplitter, Knochenmehl oder ähnliches schnell einwachsen und die intersomatische Fusion fördern. Dabei erhält das Implantat die Lordose der Wirbelsäule bzw. stellt diese wieder ein, damit die Wirbelsäule ihre natürliche Krümmung aufrecht erhalten kann.

Die Verwendung des erfindungsgemäßen Implantats ist nicht auf bestimmte Bereiche der Wirbelsäule beschränkt, vielmehr kann das Implantat von den Halswirbeln bis zu den Lendenwirbeln eingesetzt werden, wobei lediglich eine an die Größe der zu fusionierenden Wirbeln angepaßte Skalierung des Implantates erfolgen muß. Da aber nicht nur die Größe des Zwischenwirbelraumes variiert, sondern auch der von benachbarten Wirbeln eingeschlossene Winkel, ist vorgesehen, daß der Neigungswinkel eine Größe zwischen 5° und 15° aufweist.

Im Rahmen der Erfindung hat es sich als günstig erwiesen, wenn die Streben mit Abstand von den dem Wirbel zugewandten Rand der ersten Käfigplatte und/oder der zweiten Käfigplatte an dieser angeschlossen sind. Bei dieser Ausführungsform werden die erste und die zweite Käfigplatte in einem vorgegebenen Abstand zueinander fixiert, der so gewählt ist, daß die Wirbel an den einander gegenüberliegenden Enden abgestützt sind. Da die Streben einen Abstand von dem Rand der ersten und zweiten Käfigplatte einhalten, liegen diese auch nicht unmittelbar an den Deckplatten der Wirbelkörper an, sondern können in das Körpergewebe einwachsen, das durch die Streben zusätzlich stabilisiert wird und so zu einer größeren Belastbarkeit des betreffenden Wirbelsäulensegmentes führt.

In einer besonders bevorzugten Ausführungsform der Erfindung ist vorgesehen, daß die erste Käfigplatte und/oder die zweite Käfigplatte auf der dem Wirbel zugewandten Seite eine konvex gebogene Kante aufweist. Die konvex gebogene Kante ermöglicht eine Anpassung an die gebogene Form der Deckplatte des Wirbelkörpers, so daß eine über die Breite des Wirbelkörpers reichende Abstützung und nicht nur eine punktuelle Belastung erfolgt, die zu einem Einbrechen der Deckplatte führen könnte.

Um eine Fixierung des Implantats in dem Zwischenwirbelraum zu erleichtern, sind an den den Wirbeln anliegenden Flächen zur Verankerung Haltezähne ausgebildet.

Wenn das Implantat aus Titan oder einer Titanlegierung besteht, besteht weiterhin die Möglichkeit, daß das Implantat durch Röntgenaufnahmen oder durch Kernspintomographie überwacht und kontrolliert wird.

Im folgenden soll die Erfindung an einem in der Zeichnung dargestellten Ausführungsbeispielen näher erläutert werden; es zeigen:
- Fig. 1: eine Seitenansicht eines in den Zwischenwirbelraum zwischen zwei benachbarten Wirbeln eingesetzten Implantates,
- Fig. 2: eine Seitenansicht des isolierten Implantates mit eingezeichneter Flächennormale,
- Fig. 3: eine Sicht aus Richtung des Pfeiles III aus Fig. 1,
- Fig. 4: einen Längsschnitt durch die Wirbelkörper, und
- Fig. 5: eine perspektivische Darstellung des isolierten Implantats.

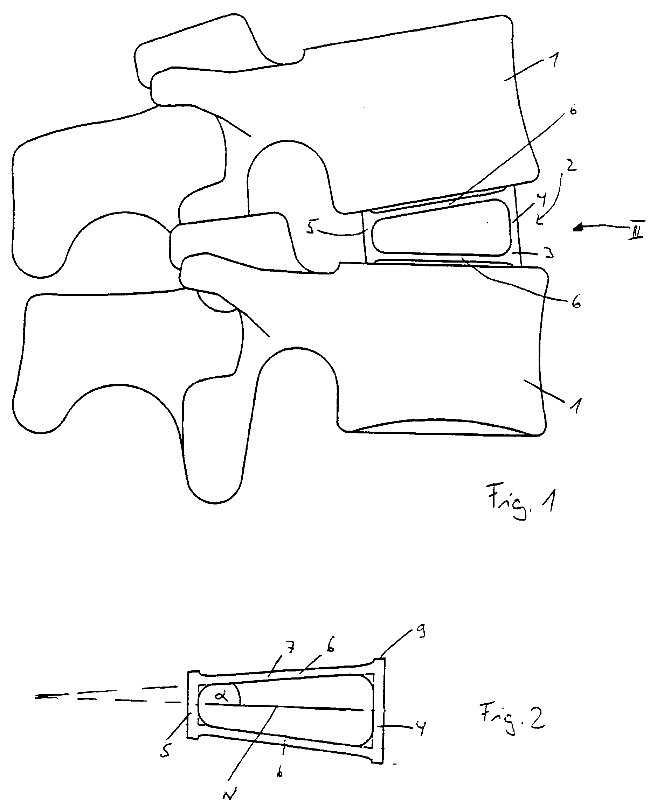

In Fig. 1 sind zwei benachbarte Wirbel 1 gezeigt, aus deren Zwischenwirbelraum 2 die Bandscheibe entfernt und ein Implantat 3 zur intersomatischen Fusion eingesetzt ist. Dies dabei gezeigte Implantat ist zur Verwendung in der ALIF (Anterior Lumbar Interbody Fusion)-Operationstechnik vorgesehen, bei der der Zugang zur Wirbelsäule anterior, vom Bauchraum aus erfolgt. Dieses Implantat 3 besteht aus einer ersten Käfigplatte 4 und aus einer zweiten Käfigplatte 5, die über Stützglieder 6 miteinander verbunden sind. Dabei ist die zweite Käfigplatte 5 kleiner als die erste Käfigplatte 4 ausgebildet. Die Stützglieder 6 sind als Streben 7 geformt und an den Eckkanten der ersten Käfigplatte und der zweiten Käfigplatte angeschlossen, so daß die Streben 7 nicht rechtwinklig mit der ersten und zweiten Käfigplatte 4,5 verbunden sind, sondern einen Neigungswinkel alpha mit deren Flächennormale N einschließen. Der Neigungswinkel alpha hat dabei eine Größe zwischen 5° und 15°, wobei der konkret zu wählende Neigungswinkel alpha von dem Einsatzort des Implantates 3 abhängt, das sowohl im Bereich der Halswirbelsäule, der Brustwirbelsäule und der Lendenwirbelsäule verwendet werden kann. Im oberen Bereich der Wirbelsäule erfolgt teilweise eine mehr zur Seite versetzte Einführung des Implantats 3 in den Zwischenwirbelraum 2.

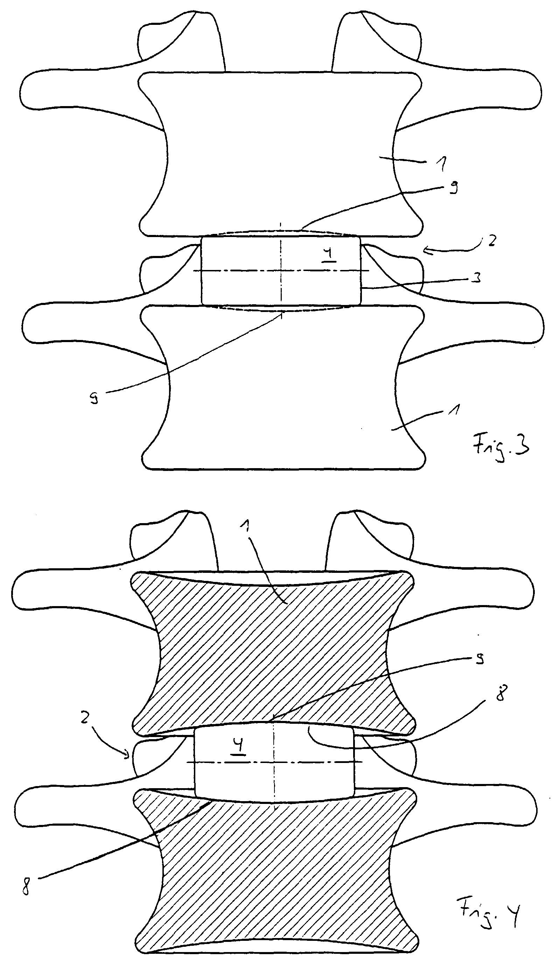

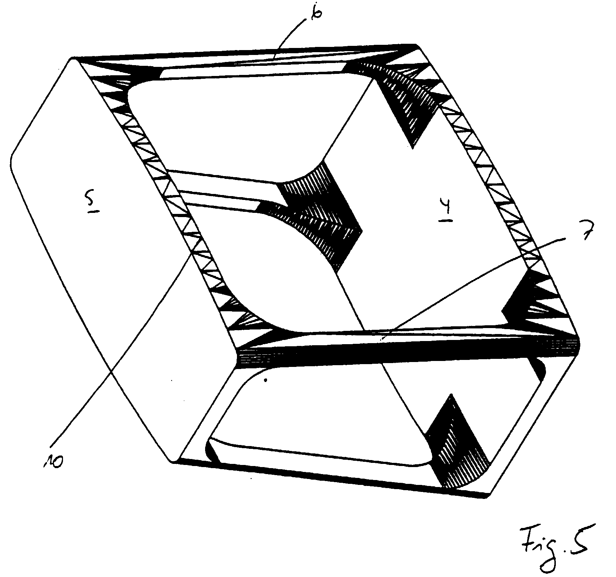

Wie sowohl aus Fig. 1 als auch Fig. 2 ersichtlich ist, sind die Streben 7 mit Abstand von dem Rand der ersten und zweiten Käfigplatte 4,5 an diesen angeschlossen, so daß die Streben 7 nicht unmittelbar an den Deckplatten 8 der Wirbelkörper anliegen, sondern gleichfalls einen Abstand einhalten. Allerdings ist es selbstverständlich im Rahmen der Erfindung nach einer nicht in der Zeichnung dargestellten Ausführungsform möglich, die Streben 7 auch unmittelbar zur Abstützung zu verwenden, so daß die Flächenpressung im Bereich der ersten und zweiten Käfigplatte 4,5 vermindert ist. Eine verminderte Flächenpressung ergibt sich auch, wenn die erste und die zweite Käfigplatte 4,5 auf der den Wirbeln 1 zugewandten Seiten eine konvex gebogene, Haltezähne 10 aufweisende Kante 9 besitzt, die der Krümmung der Deckplatten 8 der Wirbelkörper folgt und so eine über die gesamte Breite des Implantats 3 reichende Abstützung ermöglicht, wie dies besonders deutlich aus Fig. 4 zu erkennen ist. Aus Fig. 4 ist es ebenso wie aus Fig. 3 ersichtlich, daß das Implantat 3 mittig im Zwischenwirbelraum 2 sitzt und dabei eine den bei der ALIF-operationstechnik gegebenen größeren Zugang zur Wirbelsäule ausnutzende Größe aufweist, bei der ein einzelnes Implantat 3 zur Abstützung ausreicht.

Das Implantat 3 besteht aus Titan oder einer Titanlegierung, so daß das Implantat 3 leicht auf Röntgenaufnahmen oder mittels der Kernspin-Tomographie erzeugten Bilder ersichtlich ist.

## Patentansprüche

1. Implantat für die intersomatische Fusion zweier Wirbel (1) mittels eines anterior/medialen operativen Zugangs, mit einer ersten Käfigplatte (4) sowie einer über Stützglieder (6) mit der ersten Käfigplatte (4) verbundenen zweiten Käfigplatte (5), dadurch gekennzeichnet, daß die zweite Käfigplatte (5) kleiner als die erste Käfigplatte (4) ist, und daß die an den Eckkanten der ersten Käfigplatte (4) und der zweiten Käfigplatte (5) angeschlossenen, als Streben (7) ausgebildeten Stützglieder (6) einen Neigungswinkel (alpha) mit den Flächennormalen (N) der ersten Käfigplatte (4) und der zweiten Käfigplatte (5) einschließen.

2. Implantat nach Anspruch 1, dadurch gekennzeichnet, daß der Neigungswinkel (alpha) eine Größe zwischen 5° und 15° aufweist.

3. Implantat nach Anspruch 1 oder 2, dadurch gekennzeichnet, daß die Streben (7) mit Abstand von dem den Wirbeln (1) zugewandten Rand der ersten Käfigplatte (4) und/oder der zweiten Käfigplatte (5) an dieser angeschlossen sind.

4. Implantat nach einem der Ansprüche 1 bis 3, dadurch gekennzeichnet, daß die erste Käfigplatte (4) und/oder die zweite Käfigplatte (5) auf der dem Wirbel (1) zugewandten Seite eine konvex gebogene Kante (9) aufweist.

5. Implantat nach einem der Ansprüche 1 bis 4, dadurch gekennzeichnet, daß an den den Wirbeln (1) anliegenden Flächen zur Verankerung Haltezähne (10) ausgebildet sind.

6. Implantat nach einem der Ansprüche 1 bis 5, dadurch gekennzeichnet, daß es aus Titan oder einer Titanlegierung besteht.
